# EUROPEAN PATENT APPLICATION

(11) **EP 1 698 332 A1**
(43) Date of publication of application: **06.09.2006**
(21) Application number: 05090049.7
(22) Date of filing: 02.03.2005
(51) Int. Cl.: A61K 31/352, A61K 9/20, A61P 5/30

(54) **Oral modified release formulations containing 8-Prenylnaringenin for continuous estrogen support**

(71) Applicant: KAIROSmed GmbH, 10117 Berlin (DE)
(72) Inventor: Hümpel, Michael, 14163 Berlin (DE); Kranz, Heiko, 10715 Berlin (DE)
(74) Representative: Jungblut, Bernhard Jakob

(57) **Abstract**

This invention is directed to an oral modified release formulation of the phytoestrogen 8-Prenylnaringenin and its use for the treatment of symptoms of estrogen deficiency.

## Description

This invention is directed to oral modified release formulations containing 8-Prenylnaringenin (8-PN) and its use in continuous estrogen support to treat estrogen deficiency conditions.

Estrogen deficiency conditions in females can have different reasons, i.e. inactive or surgically removed ovaries or the cease of estrogen production in the menopause. One medical intervention to treat such estrogen deficiency conditions is to replace the missing estrogens (estradiol, estrone, estriol) or to use other estrogens like ethinyl estradiol or conjugated (equine) estrogens. The main use of these compounds is to replace estrogen activity in the menopause (Hormone Replacement Therapy, HRT). All these compounds are effective in the treatment of menopausal symptoms (short term) and the treatment and prevention of osteoporosis (short and long term). Recently, a new estrogen, 8-Prenylnaringenin, found in plants (hop, Anaxagorea luzolensis A. Gray) was shown to be not only the most active phyto-estrogen but also a substance that exhibits tissue specificity with a much lower activity on the uterus than estradiol at equivalent bone protective doses. On the basis of this new pharmacological profile it was claimed that menopausal symptoms and the treatment and protection of osteoporosis is possible without the concomitant administration of a progestin and thus without the induction of withdrawal bleedings (PCT/EP2004/01146). 8-Prenylnaringenin (5,7-Dihydroxy-2-(4-hydroxyphenyl)-8-(3-methylbut-2-enyl)-chroman-4-on) has the following structure:

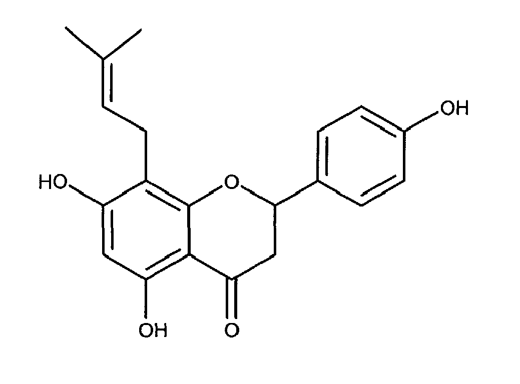

The 4-hydroxyphenyl group may either be in the 2S(-) or the 2R (+) position.

The normal route of administration in hormone replacement therapy is the oral route. This route is not only preferred because of user convenience but also because most of the substances in question need a high daily dose which is difficult to administer by alternate routes like the nasal, dermal or inhalatory routes. For the most potent natural estrogen, estradiol, the dermal route was shown to be an effective alternative to the oral route because only small quantities (25 - 100 µg/d) need to reach systemic circulation. There are two reasons why the effective daily oral dose (1 or 2 mg) can be reduced by a factor of 10 - 40 when estradiol is absorbed via the skin. Most importantly, the liver, which inactivates roughly 90 % of the oral dose before reaching the systemic circulation, is surpassed by the dermal route. Thus, avoidance of the high first-liver-pass metabolism by dermal administration leads to a drastic reduction of the effective dose. ln addition, the continuous and steady influx of drug (zero order kinetics) over the time a patch is used (2 - 7 days) has a dose sparing effect as compared to the oral route, which is typically characterised by a mixture of several first order kinetics, i.e. absorption, distribution and disposition processes starting at a definite time of the day. This leads to steep drug serum level increases shortly after intake and subsequent decreases governed by distribution and metabolism/excretion. Because all marketed estrogens, natural or synthetic, undergo an almost complete metabolisation before excretion and show high intrinsic clearance rates, drug serum levels decrease rapidly and generally drop to very low and sometimes undetectable levels within the treatment interval of 1 day. As a result, serum (and subsequently tissue) concentrations show high fluctuations with periods of maximum effects and below threshold effects. At the doses used for marketed estrogens it can be assumed that serum and tissue concentrations fell below a maximum effective level for several hours a day. It is assumed that an effective dose can be lowered by 30 - 50 % when the total dose is fairly distributed over the total treatment interval.

A potential solution to this problem could be the use of an oral modified release formulation. These pharmaceutical formulations were developed for other drugs to avoid high plasma fluctuations of the active compound.

The question, however, arises why in hormone replacement therapy no modified release formulations are available on the market. The answer is that modified release formulations need higher total doses for substances undergoing a high first-liver-pass metabolism because there is a risk that the liver would inactivate even higher parts of the total dose when it reaches the liver at small dose parts for an extended period of time. In addition, a modified release formulation can only deliver an active ingredient for the gastro-intestinal transit time, which is highly variable but takes 12 - 16 hours on an average. Thus, a modified release formulation containing one of the marketed estrogens would not be able to reach the aim to continuously replace the estrogen for 24 hours a day.

The problem, therefore, is to provide an oral formulation for 8-Prenylnaringenin which continuously distributes the 8-Prenylnaringenin for almost 24 hours a day and which preferably has to be administered only once a day.

This problem was solved by a solid oral modified release formulation of 8-Prenylnaringenin. This formulation contains a polymeric matrix, a buffer substance and one or more excipients in addition to 8-Prenylnaringenin. Preferably the buffer substance is an alkaline substance like e. g. magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof. Preferably the particle size of the compounds is in the range of 0.1 - 750 µm. Most preferably it is in the range of 20 - 400 µm. The polymer matrix is preferably chosen from the group consisting of: cellulose derivatives, acrylic derivatives, vinyl polymers, polyacrylates, polycarbonates, polyethers, polystyrenes polyanhydrides, polyesters, polyorthoesters, polysaccharides and natural polymers. Most preferably, the polymer matrix consists of water soluble polyvinylpyrrolidone or water insoluble polyvinylacetate. In a preferred embodiment the oral modified release formulation is coated with a polymeric coat.

Excipients may be chosen from the group consisting of lactose, calcium phosphate, manitol and starch. Preferably an additional excipient is microcrystalline cellulose.

The solid oral modified release formulations of this invention show a pH-independent drug release in vitro. This is important as the pH varies considerably in the gastro-intestinal tract and continuous release should be achieved independent of the pH. However, the solubility of 8-Prenylnaringenin is pH-dependent. The compound shows higher solubility at higher pH-values whereas the solubility of the compound is low at lower pH-values. The low acid solubility property of 8-Prenylnaringenin results in vitro in slow drug dissolution at pH 1, whereas the dissolution is fast at higher pH-values such as pH 6.8. The resulting dissolution profiles are different at different pH-values. This problem is solved by the solid oral modified release formulation of this invention.

The solid oral modified release formulation of this invention solves the problem of a continuous distribution of 8-Prenylnaringenin almost over 24 h. This is a combined effect of the pharmacokinetic profile of 8-Prenylnaringenin which is drastically different from those of other estrogens and the solid oral modified release formulation.

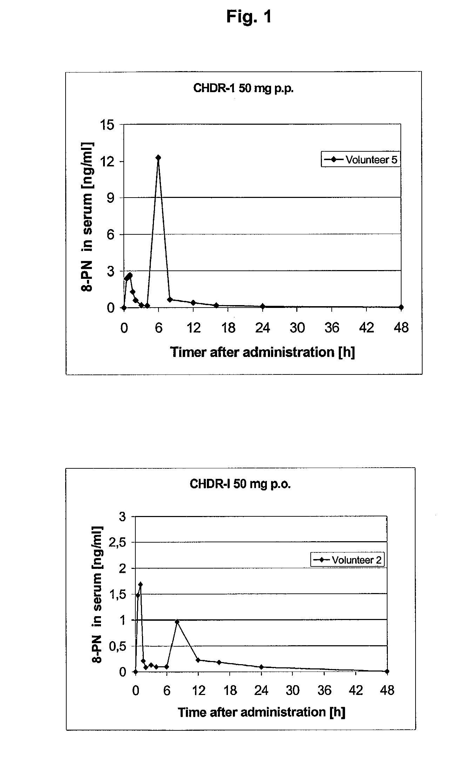

The pharmacokinetic profile of 8-Prenylnaringenin is characterized by a complete oral absorption, a low degree of metabolisation (less than 60 % of dose) and a high pre-systemic elimination (first-liver-pass excretion; about 55 % of dose). The high and unexpected pre-systemic elimination leads to a collection of substantial dose parts in the bile fluid from which these dose parts are secreted into the duodenum when gastric signaling occurs with a meal uptake. Respective dose parts are then absorbed again and reach systemic circulation. Examples for this effect (drug serum levels) are shown in Fig. 1. Data were taken from the clinical Phase la study and represent two volunteers of the lowest dose group (50 mg 8-Prenylnaringenin).

Taking the total area under the curve as a measure for 8-Prenylnaringenin systemic availability, the percentage of the area under the second peak (generated by reabsorption of the pre-systemically eliminated and then biliary secreted dose parts) can be used to estimate those dose parts which undergo pre-systemic elimination after oral dosing. On an average of six women investigated this figure is 54 ± 20 %.

The invention is to combine this unexpected pharmacokinetic property of 8-Prenylnaringenin with a modified release formulation, which can release the drug at an almost constant rate for 8 - 10 hours. The drug release from the solid oral modified release formulation according to this invention is preferably close to or perfect zero order kinetics. This oral modified release formulation is preferably taken in the evening (after dinner or before bed-time). During nighttime 8-Prenylnaringenin is released at almost constant rate leading to flat drug serum levels and a collection of substantial dose parts in the bile fluid which - after reabsorption - account for more than half of total systemically available dose. The next day these dose parts are secreted into the duodenum when the first meal (breakfast or lunch) is taken. Re-absorption gives rise to elevated 8-Prenylnaringenin serum levels over the first half of the day, which - at a lower level - repeats with dinner in the evening. Overall, the total systemically available dose is distributed over 24 hours avoiding unnecessary high peaks and ensuring effective drug concentrations in target tissues. The anticipated daily dose is between 50 and 250 mg, preferably between 75 and 150 mg.

In yet another aspect, this invention provides a method of treating a human patient who is suffering from estrogen deficiency conditions by administering a solid oral modified release 8-Prenylnaringenin formulation of the invention to the patient twice or preferably once daily. The invention is also related to the use of said oral modified release formulations for the production of a medicament for the treatment of symptoms of estrogen deficiency or for the treatment of menopausal symptoms. Said symptoms can be hot flushes, night sweat, mood disturbances or osteoporosis.

Still another aspect, this invention provides a method of maintaining therapeutically effective levels of 8-Prenylnaringenin in human plasma by administering an 8-Prenylnaringenin containing oral modified release formulation twice or preferably once daily.

The method includes administering an oral modified release formulation including from about 5 to about 80 % by weight 8-Prenylnaringenin in no more than two dosage forms per dose to the human patient, to maintain 8-Prenylnaringenin plasma levels in the human patient from about 0.5 to about 5 ng 8-PN/ml for at least 24 hours wherein the dose is administered at a frequency selected from twice or preferably once a day.

The formulations of this invention may be either in the form of single unit dosages, e.g. tablets, or in the form of multiple unit dosages, e.g. granulates, pellets or mini-tablets. These multiple unit dosages may be filled in gelatine capsules or compressed to tablets.

The single unit dosages may be produced by powder blending and direct compression into tablets or by powder blending, granulation and compression into tablets. The tablets may be coated by a film.

Multiple unit dosage may be produced by extrusion/spheronization, by layering technique, by rotor granulation, by powder blending and direct compression into mini tablets, by powder blending, granulation and compression into mini tablets, by powder blending, direct compression into mini tablets and film coating or by powder blending, granulation, compression into mini tablets and film coating.

8-Prenylnaringenin can be produced by the method described in PCT/EP2004/01146.

### Description of the figures

Fig. 1 shows drug serum levels following a single oral dose of 50 mg 8-Prenylnaringenin in two postmenopausal women; 8-Prenylnaringenin was administered as a 1:1 mixture with lactose (gelatine capsule) at fasted state in the morning, lunch was served 6 hours later. Re-increases in drug serum levels show re-absorption of dose parts collected in the bile fluid and subsequently secreted triggered by lunch.

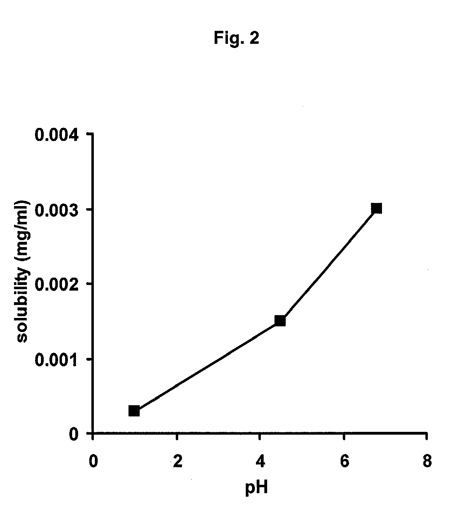

Fig. 2 shows the pH-dependent solubility of 8-Prenylnaringenin.

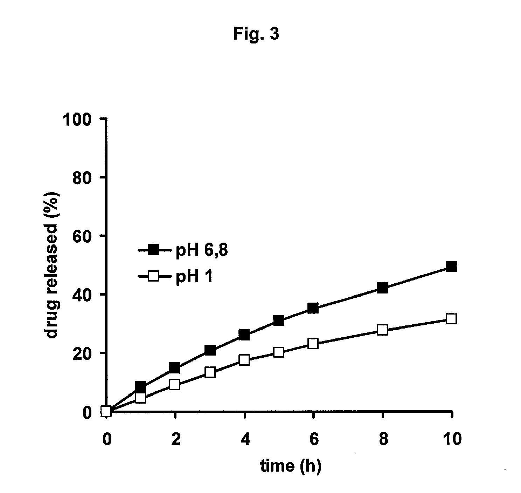

Fig. 3 shows the pH-dependent release of 8-Prenylnaringenin from mini matrix tablets prepared without buffer substance (according to Example 1).

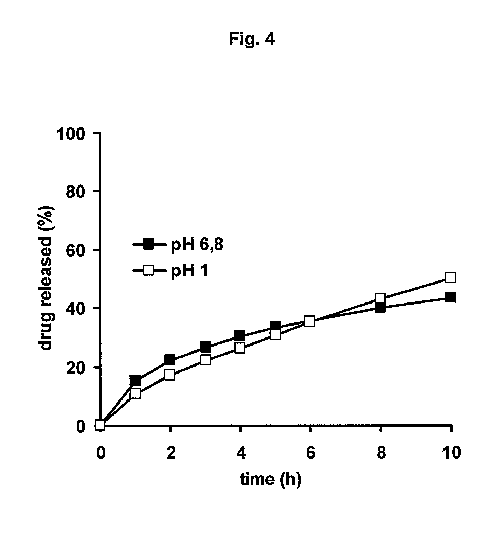

Fig. 4 shows the pH-independent release of 8-Prenylnaringenin from mini matrix tablets prepared after the addition of magnesium oxide (according to Example 2).

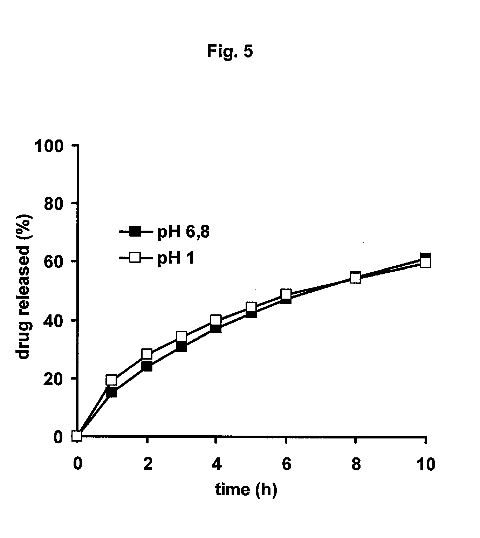

Fig. 5 shows the pH-independent release of 8-Prenylnaringenin from mini matrix tablets prepared after the addition of magnesium hydroxide (according to Example 3).

### Examples:

### Example 1

Preparation of a modified release formulation of 8-Prenylnaringenin

### Production of Mini-Matrix Tablets by Means of Direct Tableting

2.333 mg of 8-Prenylnaringenin
1.000 mg of Kollidon SR®
1.992 mg of lactose
1.500 mg microcrystalline cellulose
0.070 mg of highly dispersed silicon dioxide
0.105 mg of magnesium stearate

8-Prenylnaringenin, Kollidon SR® , lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 2

### Production of Mini-Matrix Tablets by Means of Direct Tableting

2.333 mg of 8-Prenylnaringenin
1.000 mg of Kollidon SR®
1.169 mg of magnesium oxide
0.823 mg of lactose
1.500 mg microcrystalline cellulose
0.070 mg of highly dispersed silicon dioxide
0.105 mg of magnesium stearate

8-Prenylnaringenin, Kollidon SR® , magnesium oxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 3

### Production of Mini-Matrix Tablets by Means of Direct Tableting

2.333 mg of 8-Prenylnaringenin
1.000 mg of Kollidon SR®
1.169 mg of magnesium hydroxide
0.823 mg of lactose
1.500 mg microcrystalline cellulose
0.070 mg of highly dispersed silicon dioxide
0.105 mg of magnesium stearate

8-Prenylnaringenin, Kollidon SR® , magnesium hydroxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 4

### Measurement of the Release of 8-Prenylnaringenin

Measurement of the active ingredient release from mini-matrix tablets is carried out according to a one-compartment method (basket apparatus), as described in U.S. Pharmacopeia USP XXV. The release of 8-Prenylnaringenin was examined in phosphate buffer solution, pH 6.8 (composition, see USP XXV) or in 0.1 N HCl. Ten percent (w/w) hydroxypropyl-β-cyclodextrine were added in order to achieve sink conditions and primarily control the drug release by the dosage form.

### Example 5

### Method for quantitative analysis of 8-PN in biological matrices

8-Prenylnaringenin is analyzed by a specifically developed radio-immunoassay. A 4'-O hapten ({4-[5,7-Dihydroxy-8-(3-methyl-but-2-enyl)-4-oxo-chroman-2-yl]-phenoxy}-acetic acid) was synthesized starting from racemic naringenin and coupled to cationized bovine serum albumin (cBSA). This antigen was mixed with Freund's adjuvans and injected into rabbits. The antiserum resulting after several boosters was isolated as IgG fraction and used in a final dilution of 1:100.000. A tritiated tracer was synthesized by preparation of the 3',5'-dibromo derivative of 8-Prenylnaringenin again starting from racemic naringenin. Both bromine atoms were exchanged to ³H in a final, palladium-catalyzed step leading to the tritiated tracer of a specific radioactivity of 2.22 GBq/mg.

Biological matrices, i.e. blood plasma, blood serum, urine are either directly extracted with tert.BME or after enzymatic de-conjugation by means of glucuronidase/arylsulphatase (helix pomatia). Organic layers are separated, evaporated and the residues taken up in assay buffer. Extract residues or dilutions of standard 8-PN solutions are then mixed with antiserum and tracer and kept at 4°C overnight. Dextran coated charcoal is added to separate bound and free 8-Prenylnaringenin and bound radioactivity is quantified with a liquid scintillation counter after addition of scintillation cocktail.

## Claims

1. Oral modified release formulation containing 8-Prenylnaringenin, a polymeric matrix, a buffer substance and one or more excipients.

2. Oral modified release formulation containing 8-Prenylnaringenin, a polymeric matrix, a buffer substance and one or more excipients and where the particle size of the compounds is in the range of 0.1 - 750 µm.

3. Oral modified release formulation containing 8-Prenylnaringenin according to claim 1 or 2 wherein the buffer substance is an alkaline substance.

4. Oral modified release formulation containing 8-Prenylnaringenin according to claims 1, 2 or 3 wherein said formulation is coated with a polymeric coat that affects the dissolution of 8-Prenylnaringenin.

5. Oral modified release formulation according to claims 1 - 4 wherein the polymer matrix is chosen from the group of the following materials: cellulose derivatives, acrylic derivatives, vinyl polymers, polyacrylates, polycarbonates, polyethers, polystyrenes polyanhydrides, polyesters, polyorthoesters, polysaccharides and natural polymers.

6. Oral modified release formulation according to claims 1 - 4 wherein the polymer matrix is chosen from water soluble polyvinylpyrrolidone and water insoluble polyvinylacetate.

7. Oral modified release formulation according to claims 1 - 6 wherein the buffer substance is magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof.

8. Oral modified release formulation according to claims 1 - 7 wherein the formulation contains an additional lubricant.

9. Oral modified release formulation according to claims 1 - 8 wherein the excipient is lactose, calcium phosphate, manitol or starch.

10. Oral modified release formulation according to claims 1 - 9 wherein the formulation contains microcrystalline cellulose as an additional excipient.

11. Oral modified release formulation according to claims 1 - 10 wherein the formulation contains silicon dioxide as flow promoter.

12. Oral modified release formulation according to claims 1-11 wherein the particle size of the powder mixtures is between 20 - 400 µm.

13. Use of the oral modified release formulation according to claims 1-12 for the production of a medicament for the treatment of symptoms of estrogen deficiency

14. Use of the oral modified release formulation according to claims 1-12 for the production of a medicament for the treatment of menopausal symptoms

15. Use of the oral modified release formulation according to claims 1-12 for the production of a medicament for the treatment of hot flushes
